# EUROPEAN PATENT SPECIFICATION

(11) **EP 2 533 785 B1**
(45) Date of publication and mention of the grant of the patent: **23.04.2014**
(21) Application number: 11707610.9
(22) Date of filing: 10.02.2011
(51) Int. Cl.: A61K 31/519, A61P 25/00

(54) **TREATMENT OF LOSS OF SENSE OF TOUCH WITH SAXITOXIN DERIVATIVES**
BEHANDLUNG DES VERLUSTS DES TASTSINNS MIT SAXITOXIN DERIVATEN
TRAITEMENT DE LA PERTE DE LA SENSE TU TOUCHER AVEC DES DÉRIVÉS DE LA SAXITOXINE

(30) Priority: 11.03.2010 US 312903 P; 10.02.2010 EP 10153234
(43) Date of publication of application: 19.12.2012
(73) Proprietor: Phytotox Limited, Hamilton HM12 (BM)
(72) Inventor: RUTMAN, Max, Santiago (CL); PILORGET, Jean Jacques, Santiago (CL); SIGALA, Constanza, Santiago (CL); VALENZUELA, Pablo, Santiago (CL)
(74) Representative: Ehnis, Tobias
(86) International application number: PCT/EP2011/051992
(87) International publication number: WO 2011/098539

(56) References cited:
- WO-A1-2005/110417
- WO-A1-2006/032481
- US-A1- 2003 100 574

## Description

The invention concerns a sodium channel blocker for the treatment of a human being or another mammal and a pharmaceutical composition comprising that sodium channel blocker as well as method of treatment.

A sodium channel blocker is a compound that specifically binds to a sodium channel in an axon of a neuron and specifically blocks the passage of sodium ions through that sodium channel.

From WO 2005/110417 A1 pharmaceutical compositions for interfering with neuronal transmission comprising an effective amount of at least one tricyclic 3,4-propinoperhydropurine and at least one additional compound are known.

From US 2003/0100574 A1 pharmaceutical compositions comprising tricyclic 3,4-propinoperhydropurines and uses thereof for blocking neuronal transmission are known.

From WO 2006/032481 A1 the use of a sodium channel blocker and/or one of its derivatives for the production of a medicament for the treatment of central-nervously derived neuropathic pain is known.

From WO 2006/032459 A1 the use of a sodium channel blocker and/or one of its derivatives for the production of a medicament for the treatment of peripheral-nervously derived neuropathic pain is known.

From WO 2007/110221 A1 the use of a sodium channel blocker and/or its derivatives for the production of a medicament for the treatment of neuropathic pain developing as a consequence of chemotherapy is known.

In many diseases such as diabetes mellitus or neuropathy the reduction or loss of superficial sensitivity or sense of touch is a problem. It may lead to painless severe infections and other painless wounds like burns or cuts of which the patients remain unaware.

The problem to be solved by the present invention is to provide a substance and a pharmaceutical composition as well as a method for the treatment of a reduction or loss of superficial sensitivity or sense of touch of a human being or another mammal.

The problem is solved by the subject-matter of claims 1 and 8. Embodiments of the invention are subject matter of claims 2 to 7 and 9 to 15.

According to the invention a sodium channel blocker (SCB) for use in the treatment of a reduction or loss of superficial sensitivity or sense of touch of a human being or another mammal is provided. The SCB is saxitoxin, tetrodotoxin or a tricyclic 3,4-propinoperhydropurine represented by the following formula (I) 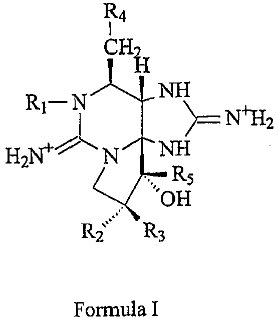 wherein R₁ and R₅ are independently selected from the group consisting of -H and -OH; R₂ and R₃ are independently selected from the group consisting of -H, -OSO₃⁻ and -SO₃; and R₄ is selected from the group consisting of -H, -OH, -OCONH₂, -OCONHSO₃⁻ and -OCOCH₃.

The sodium channel blocker may be administered over a period of between one to seven days and/or in multiple treatment cycles. By "superficial sensitivity" the ability of the human being or other mammal to register external stimuli like heat, cold or pressure on its skin or mucosa is meant. By "sense of touch" any ability of the human being or other mammal to register a touch is meant. The sense of touch is a specific superficial sensitivity. A reduction or loss of superficial sensitivity is often felt as numbness. The reduction or loss of superficial sensitivity or sense of touch is a pathological condition that may be caused by a pathogen, by a medical treatment such as chemotherapy or ray treatment, under which it may occur as side effect, by the use of weapons, or by a nuclear accident.

The inventors of the present invention have recognized that a reduction or loss of superficial sensitivity or sense of touch can be treated with an SCB according to the invention such that the superficial sensitivity or the sense of touch is at least partly restored. For example, in diabetes mellitus there is a loss of superficial sensitivity in the extremities. This loss of superficial sensitivity can be treated with an SCB according to the invention such that sensitivity will be restored.

The effect of recovery of sensitivity is independent of the presence of pain such as a neuropathic pain. This means that the effect is not just owing to a possibly also occurring suppression of pain that without treatment drowns out every other feeling. The treatment of a reduction or loss of superficial sensitivity or sense of touch is different from the known treatment of neuropathic pain with SCBs.

The effect of the treatment is also totally different from the effect of a treatment of neuropathic pain with a local anesthetic such as lidocaine which is also an SCB. If pain caused by neuropathy is treated with lidocaine, pain and sensitivity are lost in the area innervated by nerves affected by the treatment. During treatment with lidocaine the loss of sensitivity lasts as long as the pain relief. The superficial sensitivity and sense of touch restoring activity of the SCB according to the invention is a complete new effect. This effect is totally surprising because in higher dosages SCBs can totally block the propagation of action potentials along axons of neurons.

In an embodiment of the invention either one of R₂ and R₃ is -OSO₃⁻ or R₄ is -OCONHSO₃⁻. The tricyclic 3,4-propinoperhydro-purine may be one of the derivatives of saxitoxin or a gonyautoxin (hereinafter "GTX") according to formula I as set forth in the table below.

| **Compound** | **R₁** | **R₂** | **R₃** | **R₄** | **R₅** |
|---|---|---|---|---|---|
| Gonyautoxin 1 | -OH | -H | -OSO⁻₃ | -OCONH₂ | -OH |
| Gonyautoxin 2 | -H | -H | -OSO⁻₃ | -OCONH₂ | -OH |
| Gonyautoxin 3 | -H | -OSO⁻₃ | -H | -OCONH₂ | -OH |
| Gonyautoxin 4 | -OH | -OSO⁻₃ | -H | -OCONH₂ | -OH |
| Gonyautoxin 5 | -H | -H | -H | -OCONHSO₃⁻ | -OH |
| Neosaxitoxin | -OH | -H | -H | -OCONH₂ | -OH |
| Descarbamoylsaxitoxin | -OH | -H | -H | -OH | -OH |

In one embodiment the SCB according to the invention is in the form of its racemate, pure stereoisomer, especially enantiomer or diastereomer or in the form of a mixture of stereoisomers, especially enantiomers or diastereomers, in neutral form, in the form of an acid or base or in the form of a salt, especially a physiologically acceptable salt, or in the form of a solvate, especially a hydrate.

The reduction or loss of the superficial sensitivity or sense of touch may be a side effect of a drug or of a medical treatment or may be caused by diabetes mellitus, a viral infection, in particular a Herpes virus infection or a Varizella-Zoster virus infection, allodynia, causalgia, hyperalgesia, hyperesthesia, hyperpathia, neuralgia, neuritis, neuropathy or other neurologic cause.

In one embodiment the SCB according to the invention is saxitoxin, wherein the saxitoxin is synthetically synthesised or isolated from a biological source, in particular from cyanobacteria or from contaminated shellfish, especially shellfish contaminated with A. catenella.

The SCB according to the invention may also be tetrodotoxin or the tricyclic 3,4-propinoperhydropurine, wherein the tetrodotoxin, or the tricyclic 3,4-propinoperhydropurine is synthetically synthesised or isolated from a biological source. In case of tetrodotoxin the biological source may be a puffer fish.

The invention further concerns a pharmaceutical composition comprising at least one sodium channel blocker according to the invention and a pharmacologically acceptable carrier for use in the treatment of a reduction or loss of superficial sensitivity or sense of touch of a human being or another mammal. The carrier may be any material suitable for topical drug administration. Carriers include any such materials known in the art which is non-toxic in the amount used, and does not interact with other components of the composition in deleterious manner.

In an embodiment the SCB according to the invention is contained in the pharmaceutical composition in an amount suitable for an administration of 0.01 to 1000 µg, in particular 0.1 to 100 µg, especially 1 to 10 µg, SCB per day. The SCB according to the invention may be contained in the pharmaceutical composition in a concentration of 0.01 to 1000 µg per ml, in particular 0.1 to 100 µg per ml, especially 1 to 10 µg per ml.

The pharmaceutical composition according to the invention may be a pharmaceutical composition prepared for injection, in particular intramuscular, intravenous, intradermal, or subcutaneous injection, prepared for topical administration, in particular superficial administration, or prepared for systemic administration, in particular oral administration. The pharmaceutical composition prepared for superficial administration can be a skin-patch, a cream, an ointment, or a spray. The administration may be supported physically in particular by UV light, ultra sound, iontophoresis, phonophoresis, or mechanical modulation.

According to an embodiment of the invention the pharmaceutical composition further comprises at least one analgesic compound. The analgesic compound can be lidocaine, bupivacaine, fentanyl, or acetaminophen.

The SCB in the pharmaceutical composition according to the invention may be contained in a liposome or a microemulsion. A microemulsion is a stable, isotropic liquid mixture of oil, water and surfactant, frequently in combination with a cosurfactant. The mixture is an emulsion with oil dispersed in water or water dispersed in oil the dispersed phase of which is forming such small domains that visible light is not scattered by the dispersed phase. Therefore, the microemulsion is clear.

Alternatively or in addition the pharmaceutical composition comprising the SCB may further comprise at least one substance facilitating the transport of the SCB through the skin. Such substances are known in the art as permeation enhancers. The substance may be a substance selected from the group consisting of: alcohols, amines, amides, amino acids, amino acid esters, 1-substituted azacycloheptan-2-ones, pyrrolidones, terpenes, fatty acids, fatty acid esters, macrocyclic compounds, tensides, sulfoxides, liposomes, transferomes, lecithin vesicles, ethosomes, anionic, cationic and non-ionic surfactants, polyols, essential oils, dimethylsulfoxide, decylmethylsulfoxide, diethylene glycol monoethyl ether, diethylene glycol monomethyl ether, sodium laurate, sodium lauryl sulfate, cetyltrimethylammonium bromide, benzalkonium chloride, a poloxamer, polysorbate 20 (Tween 20 = polyoxyethylene sorbitan monolaurate), polysorbate 40 (Tween 40 = polyoxyethylene sorbitan monopalmitate), polysorbate 60 (Tween 60 = polyoxyethylene sorbitan monostearate), polysorbate 80 (Tween 80 = polyoxyethylene sorbitan monooleate), lecithin, 1-n-dodecylcyclazacycloheptan-2-one, ethanol, propanol, octanol, benzyl alcohol, lauric acid, oleic acid, valeric acid, isopropyl myristate, isopropyl palmitate, methylpropionate, ethyl oleate, sorbitan sesquioleate, propylene glycol, ethylene glycol, glycerol, butanediol, polyethylene glycol, polyethylene glycol monolaurate, urea, dimethylacetamide, dimethylformamide, 2-pyrrolidone, 1-methyl-2-pyrrolidone, ethanol amine, diethanol amine, triethanolamine, alkanones, salicylic acid, salicylates, citric acid and succinic acid.

The poloxamer (polyethylene-polypropylene glycol, molecular formula: HO(C₂H₄O)ₐ(C₃H₆O)_{b}(C₂H₄O)ₐH, wherein a and b are integers) is a synthetic nonionic triblock block copolymer composed of a central hydrophobic chain of polyoxypropylene (poly(propylene oxide)) flanked by two hydrophilic chains of polyoxyethylene (poly(ethylene oxide)). It is available in several types such as Poloxamer 231, Poloxamer 182, or Poloxamer 184.

### EXAMPLE

The following cream composition was used for all therapeutic applications described below:

| **Ingredient** | **Concentration** |
|---|---|
| Aqua | 70,400 |
| Persea Gratissima Oil | 6,000 |
| Propylene Glycol | 5,000 |
| Squalane | 3,500 |
| Active Ingredient | xx |
| Petrolatum | 3,500 |
| Dimethicone | 3,000 |
| PEG-20 Methyl Glucose Sesquistearate | 2,500 |
| Cetyl Acetate and Acetylated Lanolin Alcohol | 2,000 (total concentration of the mentioned ingredients) |
| Diazolinidyl Urea and Methylparaben and Propylparaben and Propylene Glycol | 1,500 (total concentration of the mentioned ingredients) |
| Glyceryl Stearate | 1,000 |
| Methyl Glucose Sesquistearate | 0,500 |
| Triethanolamine | 0,300 |
| Ozokerite | 0,300 |
| Carbomer | 0,050 |
| Acrylate(s) | 0,200 |
| Parfum | 0,150 |
| Tocopherol and Ascorbyl Palmitate and Lecithin and Glyceryl Stearate and Glyceryl Oleate and Citric Acid | 0,100 |

The concentrations in the above table are given in grams of a total of 100 g. The acrylate(s) may be C 10-30 alkyl acrylate crosspolymer(s)

### 1. Active Ingredient: 10 µg/ml of a mixture of the epimers GTX-2 and GTX-3

Three patients (2 female, 1 male) used a pharmaceutical composition according to the invention containing 10 µg/ml of a mixture of the epimers GTX-2 and GTX-3. The mixture of GTX-2 and GTX-3 was contained in liposomes in the above-specified cream composition. All three patients were AIDS patients with drug-related neuropathy. All patients were receiving a drug combination including a nucleoside reverse-transcriptase inhibitor, didanosine. It is well-known that patients on didanosine may develop toxic peripheral neuropathy, usually characterised by bilateral symmetrical distal numbness, tingling, and pain in feet and, less frequently, hands.

All three patients presented numbness and loss of superficial sensitivity in addition to an intense neuropathic pain in their inferior limbs (ankles and feet). They described the pain as the worst pain ever experienced preventing them to have a normal sleep and complained that they could not feel hot and/or cold in their feet.

The first patient (female, age: 45) presented numbness and loss of sensitivity in both her feet. She had been treated with an antidepressant drug associated with analgesics. She reported that the pain was poorly controlled with the drugs she received. She began to use the pharmaceutical composition in the numb area using it once a day then up to three times a day. She reported feeling better (i.e. a decrease in her symptoms) after using the pharmaceutical composition three times daily for three days. After one week of application she reported a complete relief of the initials symptoms.

The second patient (male, age: 51) presented numbness, loss of sensitivity and neuropathic pain in both feet and his right calf. He has been treated with antidepressant drug, analgesics and vitamins for his condition. He reported no relief of the symptoms with this treatment. He used the pharmaceutical composition in topical application on the affected areas four times a day. After four days of application he felt a decrease in the symptoms, reporting a complete relief nine days after he began to use the composition.

The third patient (female, age: 47) presented loss of sensitivity and neuropathic pain in both feet and calfs. She had been treated with anticonvulsant drug, analgesics, vitamins and acupuncture. The symptoms were so intense that she attempted to stop her AIDS medication. She used the pharmaceutical composition on her painful and numb areas three times daily. After two days of treatment she reported less pain and numbness in the affected areas and relief of symptoms ten days after she began. In the meanwhile she resumed her AIDS medications. She attempted to stop the application of the pharmaceutical composition and reported that the pain and numbness came back within a few days. Resuming the application of the pharmaceutical composition resulted in pain relief and superficial sensitivity recovery within one week.

All three patients had been treated for their conditions with different drugs or drug associations including analgesics, antidepressant or anticonvulsant drugs, vitamins, or physical treatment (acupuncture) with no or few positive results. They applied the pharmaceutical composition on the numb areas one to four times per day. They reported a decrease of the symptoms after about two to four days of use and a complete relief within seven to nine days.

A further patient (female, age: 56) used the same cream composition as specified above containing 10 µg/ml of a mixture of the epimers GTX-2 and GTX-3 contained in liposomes.

This patient's history is remarkable by the existence of a grade IIb/III cervicouterine cancer in the past years, treated with chemotherapy and radiotherapy. The patient first presented with local pain on the internal side of the right arm. This pain was described as severe and unbearable, and she reported she almost could not wear clothes. This was followed by a typical zoster distributed skin eruption of an entire dermatoma (posterior and anterior T6). The eruption resolved in about two weeks. The pain lasted about six weeks from the beginning of the clinical presentation. This patient could not be treated with antidepressant or anticonvulsivant agents since she was already treated with bupropion for a post-traumatic depression. She was given NSAID (ketoprofen 200 mg twice daily), and acetaminophen (1000 mg three times daily). No opiod-like drug (tramadol) could be used due to a patient history of intolerance to this drug. She intended to use a topical preparation of lidocaine 5% with no or poor relief of the pain.

She began to use the GTX-2 and GTX-3 containing cream composition topically about one week after the onset of the symptoms. Relief of the pain and recovery of a normal superficial sensitivity was noted between 15 and 30 min after the application, lasting for about 4 to 6 hours. She continued to use the topical GTX preparation three to four times a day with the same results. In absence of application the patient noted a recurrence of the pain.

### 2. Active Ingredient: 10 µg/ml Neosaxitoxin

A further patient (male, age: 70) used the same cream composition as specified above containing 10 µg/ml neosaxitoxin contained in liposomes.

This patient has COPD (Chronic Obstructive Pulmonary Disease) of several years of evolution. Its current treatment includes prednisone (corticoid) 5 mg per day, plus inhalers. The patient presented a typical ophthalmic herpes zoster on the right side. The pain was severe and unbearable. He was given NSAID and acetaminophen because no opiods and opiods-like drugs could be used due to his respiratory condition. He began to apply the neosaxitoxin containing cream composition topically three days after the onset of the symptoms. He used it three to four times a day obtaining a relief of the pain and a recovery of the superficial sensitivity within half an hour after the application. He used the preparation during two weeks.

## Claims

1. Sodium channel blocker (SCB) for use in the treatment of a reduction or loss of superficial sensitivity or sense of touch of a human being or another mammal, wherein the SCB is saxitoxin, tetrodotoxin or a tricyclic 3,4-propinoperhydropurine represented by the following formula (I), 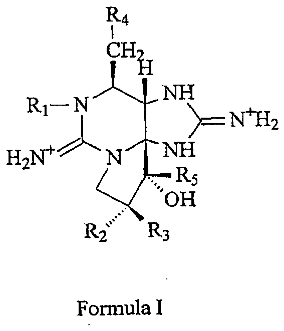 wherein R₁ and R₅ are independently selected from the group consisting of -H and -OH; R₂ and R₃ are independently selected from the group consisting of -H, -OSO₃⁻ and -SO₃; and R₄ is selected from the group consisting of -H, -OH, -OCONH₂, -OCONHSO₃⁻ and -OCOCH₃.

2. The SCB according to claim 1 for use in the treatment of a reduction or loss of superficial sensitivity or sense of touch of a human being or another mammal, wherein either one of R₂ and R₃ is -OSO₃⁻ or R₄ is -OCONHSO₃⁻.

3. The SCB according to claim 1 or 2 for use in the treatment of a reduction or loss of superficial sensitivity or sense of touch of a human being or another mammal, wherein the tricyclic 3,4-propinoperhydropurine is neosaxitoxin, descarbamoylsaxitoxin, or a gonyautoxin (GTX), in particular GTX-1, GTX-2, GTX-3, GTX-4, or GTX-5.

4. The SCB according to any of the preceding claims for use in the treatment of a reduction or loss of superficial sensitivity or sense of touch of a human being or another mammal, wherein the SCB is in the form of its racemate, pure stereoisomer, especially enantiomer or diastereomer or in the form of a mixture of stereoisomers, especially enantiomers or diastereomers, in neutral form, in the form of an acid or base or in the form of a salt, especially a physiologically acceptable salt, or in the form of a solvate, especially a hydrate.

5. The SCB according to any of the preceding claims for use in the treatment of a reduction or loss of superficial sensitivity or sense of touch of a human being or another mammal, wherein the reduction or loss of the superficial sensitivity or sense of touch is a side effect of a drug or of a medical treatment or is caused by diabetes mellitus, a viral infection, in particular a Herpes virus infection or a Varizella-Zoster virus infection, allodynia, causalgia, hyperalgesia, hyperesthesia, hyperpathia, neuralgia, neuritis, neuropathy or other neurologic cause.

6. The SCB according to any of the preceding claims for use in the treatment of a reduction or loss of superficial sensitivity or sense of touch of a human being or another mammal, wherein the SCB is saxitoxin, wherein the saxitoxin is synthetically synthesised or isolated from a biological source, in particular from cyanobacteria or from contaminated shellfish, especially shellfish contaminated with A. catenella.

7. The SCB according to any of the preceding claims for use in the treatment of a reduction or loss of superficial sensitivity or sense of touch of a human being or another mammal, wherein the SCB is tetrodotoxin or the tricyclic 3,4-propinoperhydropurine, wherein the tetrodotoxin or the tricyclic 3,4-propinoperhydropurine is synthetically synthesised or isolated from a biological source.

8. Pharmaceutical composition comprising at least one SCB according to any of the preceding claims and a pharmacologically acceptable carrier for use in the treatment of a reduction or loss of superficial sensitivity or sense of touch of a human being or another mammal.

9. The pharmaceutical composition according to claim 8 for use in the treatment of a reduction or loss of superficial sensitivity or sense of touch of a human being or another mammal, wherein the SCB is contained in an amount suitable for an administration of 0.01 to 1000 µg, in particular 0.1 to 100 µg, especially 1 to 10 µg, SCB per day.

10. The pharmaceutical composition according to claim 8 or 9 for use in the treatment of a reduction or loss of superficial sensitivity or sense of touch of a human being or another mammal, wherein the SCB is contained in a concentration of 0.01 to 1000 µg per ml, in particular 0.1 to 100 µg per ml, especially 1 to 10 µg per ml.

11. The pharmaceutical composition according to any of claims 8 to 10 for use in the treatment of a reduction or loss of superficial sensitivity or sense of touch of a human being or another mammal, wherein the pharmaceutical composition is prepared for injection, in particular intramuscular, intravenous, intradermal, or subcutaneous injection, prepared for topical administration, in particular superficial administration, or prepared for systemic administration, in particular oral administration.

12. The pharmaceutical composition according to claim 11 for use in the treatment of a reduction or loss of superficial sensitivity or sense of touch of a human being or another mammal, wherein the pharmaceutical composition prepared for superficial administration is a skin-patch, a cream, an ointment, or a spray.

13. The pharmaceutical composition according to any of claims 8 to 12 for use in the treatment of a reduction or loss of superficial sensitivity or sense of touch of a human being or another mammal, wherein the pharmaceutical composition further comprises at least one analgesic compound.

14. The pharmaceutical composition according to claim 13 for use in the treatment of a reduction or loss of superficial sensitivity or sense of touch of a human being or another mammal, wherein the analgesic compound is lidocaine, bupivacaine, fentanyl, or acetaminophen.

15. The pharmaceutical composition according to any of claims 8 to 14 for use in the treatment of a reduction or loss of superficial sensitivity or sense of touch of a human being or another mammal, wherein the SCB is contained in a liposome or a microemulsion and/or wherein the pharmaceutical composition further comprises at least one substance facilitating the transport of the SCB through the skin, in particular a substance selected from the group consisting of: alcohols, amines, amides, amino acids, amino acid esters, 1-substituted azacycloheptan-2-ones, pyrrolidones, terpenes, fatty acids, fatty acid esters, macrocyclic compounds, tensides, sulfoxides, liposomes, transferomes, lecithin vesicles, ethosomes, anionic, cationic and non-ionic surfactants, polyols, essential oils, dimethylsulfoxide, decylmethylsulfoxide, diethylene glycol monoethyl ether, diethylene glycol monomethyl ether, sodium laurate, sodium lauryl sulfate, cetyltrimethylammonium bromide, benzalkonium chloride, a poloxamer, polysorbate 20 (polyoxyethylene sorbitan monolaurate), polysorbate 40 (polyoxyethylene sorbitan monopalmitate), polysorbate 60 (polyoxyethylene sorbitan monostearate), polysorbate 80 (polyoxyethylene sorbitan monooleate), lecithin, 1-n-dodecylcycla-zacycloheptan-2-one, ethanol, propanol, octanol, benzyl alcohol, lauric acid, oleic acid, valeric acid, isopropyl myristate, isopropyl palmitate, methylpropionate, ethyl oleate, sorbitan sesquioleate, propylene glycol, ethylene glycol, glycerol, butanediol, polyethylene glycol, polyethylene glycol monolaurate, urea, dimethylacetamide, dimethylformamide, 2-pyrrolidone, 1-methyl-2-pyrrolidone, ethanol amine, diethanol amine, triethanolamine, alkanones, salicylic acid, salicylates, citric acid and succinic acid.

## Patentansprüche

1. Natriumkanalblocker (NKB) zur Verwendung bei der Behandlung einer Verminderung oder eines Verlusts von Oberflächenempfindlichkeit oder Tastsinn eines Menschen oder anderen Säugetiers, wobei der NKB Saxitoxin, Tetrodotoxin oder ein trizyklisches 3,4-Propinoperhydropurin ist, welches durch die folgende Formel (I) dargestellt ist, 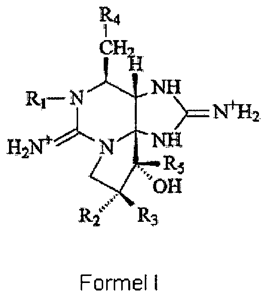 wobei R₁ und R₅ unabhängig voneinander aus der Gruppe bestehend aus -H und -OH ausgewählt sind; R₂ und R₃ unabhängig voneinander aus der Gruppe bestehend aus -H, -OSO₃⁻ und -SO₃ ausgewählt sind; und R₄ aus der Gruppe bestehend aus -H, -OH, -OCONH₂, -OCONHSO₃⁻ und -OCOCH₃ ausgewählt ist.

2. NKB nach Anspruch 1 zur Verwendung bei der Behandlung einer Verminderung oder eines Verlusts von Oberflächenempfindlichkeit oder Tastsinn eines Menschen oder eines anderen Säugetiers, wobei entweder R₂ oder R₃ -OSO₃⁻ ist oder R₄ -OCONHSO₃⁻ ist.

3. NKB nach Anspruch 1 oder 2 zur Verwendung bei der Behandlung einer Verminderung oder eines Verlusts von Oberflächenempfindlichkeit oder Tastsinn eines Menschen oder eines anderen Säugetiers, wobei das trizyklische 3,4-Propinoperhydropurin Neosaxitoxin, Descarbamoylsaxitoxin oder ein Gonyautoxin (GTX), insbesondere GTX-1, GTX-2, GTX-3, GTX-4 oder GTX-5, ist.

4. NKB nach einem der vorhergehenden Ansprüche zur Verwendung bei der Behandlung einer Verminderung oder eines Verlusts von Oberflächenempfindlichkeit oder Tastsinn eines Menschen oder eines anderen Säugetiers, wobei der NKB in der Form seines Racemats, reinen Stereoisomers, besonders Enantiomers oder Diastereomers, oder in der Form einer Mischung von Stereoisomeren, besonders Enantiomeren oder Diastereomeren, in neutraler Form, in der Form einer Säure oder Base oder in der Form eines Salzes, besonders eines physiologisch akzeptierbaren Salzes, oder in der Form eines Solvats, besonders eines Hydrats, vorliegt.

5. NKB nach einem der vorhergehenden Ansprüche zur Verwendung bei der Behandlung einer Verminderung oder eines Verlusts von Oberflächenempfindlichkeit oder Tastsinn eines Menschen oder eines anderen Säugetiers, wobei die Verminderung oder der Verlust der Oberflächenempfindlichkeit oder des Tastsinns eine Nebenwirkung eines Medikaments oder einer medizinischen Behandlung ist oder durch Diabetes mellitus, eine virale Infektion, insbesondere eine Herpesviren-Infektion oder eine Varizella-Zoster-Virusinfektion, Allodynie, Kausalgie, Hyperalgesie, Hyperästhesie, Hyperpathie, Neuralgie, Neuritis, Neuropathie oder eine andere neurologische Ursache verursacht ist.

6. NKB nach einem der vorhergehenden Ansprüche zur Verwendung bei der Behandlung einer Verminderung oder eines Verlusts von Oberflächenempfindlichkeit oder Tastsinn eines Menschen oder eines anderen Säugetiers, wobei der NKB Saxotoxin ist, wobei das Saxotoxin synthetisch synthetisiert ist oder aus einer biologischen Quelle, insbesondere aus Cyanobakterien oder aus kontaminierten Schalentieren, besonders aus mit A. catenella kontaminierten Schalentieren, isoliert ist.

7. NKB nach einem der vorhergehenden Ansprüche zur Verwendung bei der Behandlung einer Verminderung oder eines Verlusts von Oberflächenempfindlichkeit oder Tastsinn eines Menschen oder eines anderen Säugetiers, wobei der NKB Tetrodotoxin oder das trizyklische 3,4-Propinoperhydropurin ist, wobei das Tetrodotoxin oder das trizyklische 3,4-Propinoperhydropurin synthetisch synthetisiert oder aus einer biologischen Quelle isoliert ist.

8. Pharmazeutische Zusammensetzung, umfassend zumindest einen NKB nach einem der vorhergehenden Ansprüche und einen pharmakologisch akzeptierbaren Träger zur Verwendung bei der Behandlung einer Verminderung oder eines Verlusts von Oberflächenempfindlichkeit oder Tastsinn eines Menschen oder eines anderen Säugetiers.

9. Pharmazeutische Zusammensetzung nach Anspruch 8 zur Verwendung bei der Behandlung einer Verminderung oder eines Verlusts von Oberflächenempfindlichkeit oder Tastsinn eines Menschen oder eines anderen Säugetiers, wobei der NKB in einer Menge enthalten ist, welche zur Verabreichung von 0,01 bis 1000 µg, insbesondere 0,1 bis 100 µg, besonders 1 bis 10 µg, NKB pro Tag geeignet ist.

10. Pharmazeutische Zusammensetzung nach Anspruch 8 oder 9 zur Verwendung bei der Behandlung einer Verminderung oder eines Verlusts von Oberflächenempfindlichkeit oder Tastsinn eines Menschen oder eines anderen Säugetiere, wobei der NKB in einer Konzentration von 0,01 bis 1000 µg pro ml, insbesondere 0,1 bis 100 µg pro ml, besonders 1 bis 10 µg pro ml, enthalten ist.

11. Pharmazeutische Zusammensetzung nach einem der Ansprüche 8 bis 10 zur Verwendung bei der Behandlung einer Verminderung oder eines Verlusts von Oberflächenempfindlichkeit oder Tastsinn eines Menschen oder eines anderen Säugetiers, wobei die pharmazeutische Zusammensetzung zur Injektion, insbesondere zur intramuskularen, intravenösen, intradermalen oder subkutanen Injektion, zubereitet ist, zur topischen Verabreichung, insbesondere oberflächlichen Verabreichung, zubereitet ist, oder zur systemischen Verabreichung, insbesondere zur oralen Verabreichung, zubereitet ist.

12. Pharmazeutische Zusammensetzung nach Anspruch 11 zur Verwendung bei der Behandlung einer Verminderung oder eines Verlusts von Oberflächenempfindlichkeit oder Tastsinn eines Menschen oder eines anderen Säugetiers, wobei die zur oberflächlichen Verabreichung zubereitete pharmazeutische Zusammensetzung ein Hautpflaster, eine Creme, eine Salbe oder ein Spray ist.

13. Pharmazeutische Zusammensetzung nach einen der Ansprüche 8 bis 12 zur Verwendung bei der Behandlung einer Verminderung oder eines Verlusts von Oberflächenempfindlichkeit oder Tastsinn eines Menschen oder eines anderen Säugetiers, wobei die pharmazeutische Zusammensetzung weiterhin zumindest eine analgetische Verbindung umfasst.

14. Pharmazeutische Zusammensetzung nach Anspruch 13 zur Verwendung bei der Behandlung einer Verminderung oder eines Verlusts von Oberflächenempfindlichkeit oder Tastsinn eines Menschen oder eines anderen Säugetiers, wobei die analgetische Verbindung Lidocain, Bupivacain, Fentanyl oder Acetaminophen ist.

15. Pharmazeutische Zusammensetzung nach einem der Ansprüche 8 bis 14 zur Verwendung bei der Behandlung einer Verminderung oder eines Verlusts von Oberflächenempfindlichkeit oder Tastsinn eines Menschen oder eines anderen Säugetiers, wobei der NKB in einem Liposom oder einer Mikroemulsion enthalten ist und/oder wobei die pharmazeutische Zusammensetzung weiterhin zumindest eine Substanz umfasst, welche den Transport des NKBs durch die Haut erleichtert, insbesondere eine Substanz ausgewählt aus der Gruppe bestehend aus: Alkohole, Amine, Amide, Aminosäuren, Aminosäureester, 1-substituierte Azacycloheptan-2-one, Pyrrolidone, Terpene, Fettsäuren, Fettsäureester, makrozyklische Verbindungen, Tenside, Sulfoxide, Liposome, Transferome, Lecithinvesikel, Ethosome, anionische, kationische und nicht-ionische Tenside, Polyole, ätherische Öle, Dimethylsulfoxid, Decylmethylsulfoxid, Diethylenglycolmonoethylether, Diethylenglykolmonomethylether, Natriumlaurat, Natriumlaurylsulfat, Cetyltrimethylammoniumbromid, Benzalkoniumchlorid, ein Poloxamer, Polysorbat 20 (Polyoxyethylensorbitanmonolaurat), Polysorbat 40 (Polyoxyethylensorbitanmonopalmitat), Polysorbat 60 (Polyoxyethylensorbitanmonostearat), Polysorbat 80 (Polyoxyethylensorbitanmonooleat), Lecithin, 1-n-Dodecylcyclazacycloheptan-2-on, Ethanol, Propanol, Octanol, Benzylalkohol, Laurinsäure, Ölsäure, Valeriansäure, Isopropylmyristat, Isopropylpalmitat, Methylpropionat, Ethyloleat, Sorbitansesquioleat, Propylenglycol, Ethylenglycol, Glycerin, Butandiol, Polyethylenglycol, Polyethylenglycolmonolaurat, Harnstoff, Dimethylacetamid, Dimethylformamid, 2-Pyrrolidon, 1-Methyl-2-pyrrolidon, Ethanolamin, Diethanolamin, Triethanolamin, Alkanone, Salizylsäure, Salicylate, Zitronensäure und Bernsteinsäure.

## Revendications

1. Bloqueur de canal sodique (SCB) destiné à être utilisé dans le traitement d'une réduction ou perte de sensibilité superficielle ou du sens du toucher d'un être humain ou autre mammifère, dans lequel le SCB est la sanitoxine, tétrodotoxine ou une 3,4-propinoperhydropurine tricyclique représentée par la formule suivante (I) 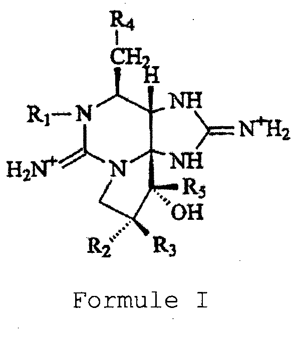 dans laquelle R₁ et R₅ sont indépendamment sélectionnés parmi le groupe constitué de -H ou -OH ; R₂ et R₃ sont indépendamment sélectionnés parmi le groupe constitué de -H, -OSO₃⁻ et -SO₃ ; et R₄ est sélectionné parmi le groupe constitué de -H, -OH, -OCONH₂, -OCONHSO₃⁻ et -OCOCH₃.

2. SCB selon la revendication 1 destiné à être utilisé dans le traitement d'une réduction ou perte de sensibilité superficielle ou du sens du toucher d'un être humain ou autre mammifère, dans lequel l'un ou l'autre de R₂ et R₃ est -OSO₃⁻ou R₄ est -OCONHSO₃⁻.

3. SCB selon la revendication 1 ou 2 destiné à être utilisé dans le traitement d'une réduction ou perte de sensibilité superficielle ou du sens du toucher d'un être humain ou autre mammifère, dans lequel la 3,4-propinoperhydropurine tricyclique est la néosaxitoxine, descarbamoylsaxitoxine ou une gonyautoxine (GTX), en particulier GTX-1, GTX-2, GTX-3, GTX-4 ou GTX-5.

4. SCB selon l'une quelconque des revendications précédentes destiné à être utilisé dans le traitement d'une réduction ou perte de sensibilité superficielle ou du sens du toucher d'un être humain ou autre mammifère, dans lequel le SCB est sous la forme de son racémate, stéréoisomère pur, notamment énantiomère ou diastéréomère ou sous la forme d'un mélange de stéréoisomères, notamment énantiomères ou diastéréomères, sous forme neutre, sous la forme d'un acide ou d'une base ou sous la forme d'un sel, notamment un sel physiologiquement acceptable, ou sous la forme d'un solvate, notamment un hydrate.

5. SCB selon l'une quelconque des revendications précédentes destiné à être utilisé dans le traitement d'une réduction ou perte de sensibilité superficielle ou du sens du toucher d'un être humain ou autre mammifère, dans lequel la réduction ou perte de sensibilité superficielle ou du sens du toucher est un effet secondaire d'un médicament ou d'un traitement médical ou est provoquée par un diabète sucré, une infection virale, en particulier une infection par le virus de l'herpès ou une infection par le virus varicelle-zona, l'allodynie, la causalgie, l'hyperalgésie, l'hyperesthésie, l'hyperpathie, la névralgie, la névrite, la neuropathie ou toute cause neurologique.

6. SCB selon l'une quelconque des revendications précédentes destiné à être utilisé dans le traitement d'une réduction ou perte de sensibilité superficielle ou du sens du toucher d'un être humain ou autre mammifère, dans lequel le SCB est la saxitoxine, dans lequel la saxitoxine est synthétisée de manière synthétique ou isolée à partir d'une source biologique, en particulier de cyanobactéries ou de crustacés contaminés, notamment de crustacés contaminés par A. catenella.

7. SCB selon l'une quelconque des revendications précédentes destiné à être utilisé dans le traitement d'une réduction ou perte de sensibilité superficielle ou du sens du toucher d'un être humain ou autre mammifère, dans lequel le SCB est la tétrodotoxine ou la 3,4-propinoperhydropurine tricyclique, dans lequel la tétrodotoxine ou la 3,4-propinoperhydropurine tricyclique est synthétisée de manière synthétique ou isolée à partir d'une source biologique.

8. Composition pharmaceutique comprenant au moins un SCB selon l'une quelconque des revendications précédentes et un support pharmacologiquement acceptable destinée à être utilisée dans le traitement d'une réduction ou perte de sensibilité superficielle ou du sens du toucher d'un être humain ou autre mammifère.

9. Composition pharmaceutique selon la revendication 8 destinée à être utilisée dans le traitement d'une réduction ou perte de sensibilité superficielle ou du sens du toucher d'un être humain ou autre mammifère, dans laquelle le SCB est contenu en une quantité convenant à une administration de 0,01 à 1000 µg, en particulier 0,1 à 100 µg, notamment 1 à 10 µg, de SCB par jour.

10. Composition pharmaceutique selon la revendication 8 ou 9 destinée à être utilisée dans le traitement d'une réduction ou perte de sensibilité superficielle ou du sens du toucher d'un être humain ou autre mammifère, dans laquelle le SCB est contenu en une concentration de 0,01 à 1000 µg par ml, en particulier 0,1 à 100 µg par ml, notamment 1 à 10 µg par ml.

11. Composition pharmaceutique selon l'une quelconque des revendications 8 à 10 destinée à être utilisée dans le traitement d'une réduction ou perte de sensibilité superficielle ou du sens du toucher d'un être humain ou autre mammifère, dans laquelle la composition pharmaceutique est préparée pour une injection, en particulier une injection intramusculaire, intraveineuse, intradermique ou sous-cutanée, préparée pour une administration topique, en particulier une administration superficielle, ou préparée pour une administration systémique, en particulier une administration orale.

12. Composition pharmaceutique selon la revendication 11 destinée à être utilisée dans le traitement d'une réduction ou perte de sensibilité superficielle ou du sens du toucher d'un être humain ou autre mammifère, dans laquelle la composition pharmaceutique préparée pour une administration superficielle est un timbre transdermique, une crème, une pommade ou un spray.

13. Composition pharmaceutique selon l'une quelconque des revendications 8 à 12 destinée à être utilisée dans le traitement d'une réduction ou perte de sensibilité superficielle ou du sens du toucher d'un être humain ou autre mammifère, dans laquelle la composition pharmaceutique comprend en outre au moins un composé analgésique.

14. Composition pharmaceutique selon la revendication 13 destinée à être utilisée dans le traitement d'une réduction ou perte de sensibilité superficielle ou du sens du toucher d'un être humain ou autre mammifère, dans laquelle le composé analgésique est la lidocaïne, la bupivacaïne, le fentanyl ou l'acétaminophène.

15. Composition pharmaceutique selon l'une quelconque des revendications 8 à 14 destinée à être utilisée dans le traitement d'une réduction ou perte de sensibilité superficielle ou du sens du toucher d'un être humain ou autre mammifère, dans laquelle le SCB est contenu dans un liposome ou une microémulsion et/ou dans lequel la composition pharmaceutique comprend en outre au moins une substance facilitant le transport du SCB à travers la peau, en particulier une substance sélectionnée parmi le groupe constitué de : alcools, amines, amides, acides aminés, esters d'acide aminé, azacycloheptan-2-ones 1-substituées, pyrrolidones, terpènes, acides gras, esters d'acide gras, composés macrocycliques, tensioactifs, sulfoxydes, liposomes, transféromes, vésicules de lécithine, éthosomes, tensioactifs anioniques, cationiques et non ioniques, polyols, huiles essentielles, diméthylsulfoxyde, décylméthylsulfoxyde, éther diéthylèneglycolmonoéthylique, éther diéthylèneglycolmonométhylique, laurate sodique, laurylsulfate sodique, bromure de cétyltriméthylammonium, chlorure de benzalkonium, un poloxamère, polysorbate 20 (monolaurate de polyoxyéthylène sorbitane), polysorbate 40 (monopalmitate de polyoxyéthylène sorbitane), polysorbate 60 (monostéarate de polyoxyéthylène sorbitane), polysorbate 80 (monooléate de polyoxyéthylène sorbitane), lécithine, 1-n-dodécylcyclazacycloheptan-2-one, éthanol, propanol, octanol, alcool benzylique, acide laurique, acide oléique, acide valérique, myristate isopropylique, palmitate isopropylique, méthylpropionate, oléate éthylique, sesquioléate de sorbitane, propylèneglycol, éthylèneglycol, glycérol, butanediol, polyéthylèneglycol, monolaurate de polyéthylèneglycol, urée, diméthylacétamide, diméthylformamide, 2-pyrrolidone, 1-méthyl-2-pyrrolidone, éthanolamine, diéthanolamine, triéthanolamine, alcanones, acide salicylique, salicylates, acide citrique et acide succinique.
